# EUROPEAN PATENT SPECIFICATION

(11) **EP 0 513 163 B1**
(45) Date of publication and mention of the grant of the patent: **02.01.1997**
(21) Application number: 91903968.5
(22) Date of filing: 06.02.1991
(51) Int. Cl.: A61M 16/01

(54) **SYSTEM ARRANGEMENT FOR THE EVACUATION OF ANAESTHESIA OR ANALGESIA GAS**
SYSTEMANORDNUNG ZUM EVAKUIEREN VON ANÄSTHESIE- ODER ANALGESIEGAS
AGENCEMENT DE SYSTEME POUR L'EVACUATION DE GAZ ANESTHESIANTS OU ANALGESIQUES

(30) Priority: 07.02.1990 SE 9000445
(43) Date of publication of application: 19.11.1992
(73) Proprietor: MEDICVENT AB, 902 54 Umea (SE)
(72) Inventor: LINDKVIST, Allan, S-902 53 Umea (SE)
(74) Representative: Nordén, J. Ake
(86) International application number: PCT/SE91/00082
(87) International publication number: WO 91/12043

(56) References cited:
- WO-A-82/01999
- US-A- 4 219 020
- US-A- 4 265 239
- US-A- 4 770 169

## Description

This invention relates to an apparatus of the kind indicated in the claims and more in detail to an arrangement for safeguarding of the necessary suction or evacuation effect and function at plants or systems where there is a demand for evacuation simultaneously with utilizing so called double masks or the like.

### General background

On arranging evacuation plants or systems for the evacuation of leaked out or otherwise free anaesthesia or analgesia gases it is naturally very important to design and dimension the suction devices in an appropriate way. At the same time it is urgent and important to adjust the capacity of the suction sources in such a way that the suction force remains at the desired value. The Swedish patent No 8008962-6 and European patent No. 0 067 196 disclose a known anaesthesia mask, equipped with means for evacuation of leaked out gas and the mask is so designed that it catches or collects and evacuates gas leaking out between the mask edge and the face of the patient, but is also so arranged that it is able to catch and evacuate all gas streaming out from the mask as the mask is removed from the face of the patient and is held free. For this purpose the mask proper is equipped with a circumferential slot or opening connected to a suction source. Behind the invention lies a problem which now has found a solution, viz. evacuation also of gases exhaled by the patient.

With modern anaesthesia arrangements there are besides the means for supplying of anaesthesia gas also an evacuation system, which is adapted to remove the exhaled gas mixed air directly from the mask. Thus, to the mask there are connected both a gas supply main and an evacuation main and furthermore the suction pipe or tube connected to the slot evacuation arrangement of the mask according to said patents. It is of utmost importance that the vacuum inside the evacuation main is maintained at such a level that the respiratory system of the patient in no way will be affected and this is the reason why there are built in safety devices preventing the vacuum from adversely affecting the patient. The capacity of the evacuation system is normally of a range 0,02 - 0,03 m³/h (20 - 30 litres per hour). With systems of the type described and including so called double mask devices principally no gas at all or very little gas will leak out in the operation theatre.

A known apparatus discloses a control valve having an input opening for so called fresh gas, i.e. the intended mixture of anaestesia gas and oxygen/air and to this input opening a compensation balloon is connected to an open branch pipe, which balloon in sequence with the breathing of the patient will be emptied and filled again. The evacuation line for exhaled air is also arranged at the control valve and via an open branch line connected with a so called reservoir, i.e. a flexible tank or container open towards the atmosphere, also serving as a balancing or compensation means but having as its main purpose to prevent a vacuum build up beyond the allowed limits in the evacuation main if the pipe or tube to the mask would be partially blocked.

US-A-4 265 239 discloses an apparatus of the kind similar to the one just described. In the known apparatus a so called reservoir is formed by a pipe, called exhaust pipe, at its one end open towards the ambient atmosphere and at its other end communicating with the interior of the anaesthetic apparatus via a resistance means. There is also arranged an excess gas removing device connected to a vacuum source and arranged to remove gas leaking out at the mask. In the known apparatus a tee divider, a three part flow divider means is arranged in a line between the mask gas removing means and the vacuum source, one opening of the divider being connected to the line from the mask device, one opening communicating with the source of vacuum and the third opening communicating with the reservoir or exhaust pipe of the anaesthetic apparatus in order to remove therefrom exhaled anaesthetic gas emerging through the resistance means. The vacuum in the line towards the mask device is identical to the vacuum at the branch communicating with the reservoir meaning that an unacceptably high vacuum could act inside the exhaust pipe or reservoir if the open end of the reservoir was closed.

Many hospitals and clinics, however, have no permanent systems for the supply of anaesthesy gas and the evacuation of exhaled air, but utilize portable anaesthesy apparatuses and gas bottles. With such apparatuses the exhalation normally is done directly into the operation theatre via a check valve arranged at the mask, which naturally results in a severe contamination of the air in the operating theatre and necessitates a powerful general ventilation thereof. The use of masks of the type mentioned earlier herein and securing an evacuation of leaked out gas along the edge of the mask and catching up of gas from the mask when lifted, naturally partially improves the situation, but is in no way sufficient as the exhaled air with its high anaesthesy gas contents escapes freely into the operation theatre atmosphere.

### Purpose of the invention

One object of this invention is to bring about an arrangement adaptable to make it possible to remove in the most efficient way all gas both exhaled and leaked out at the mask or otherwise escaped and to bring about a device enabling evacuation of the exhaled air without the need for specific evacuation systems.

### Description of the invention

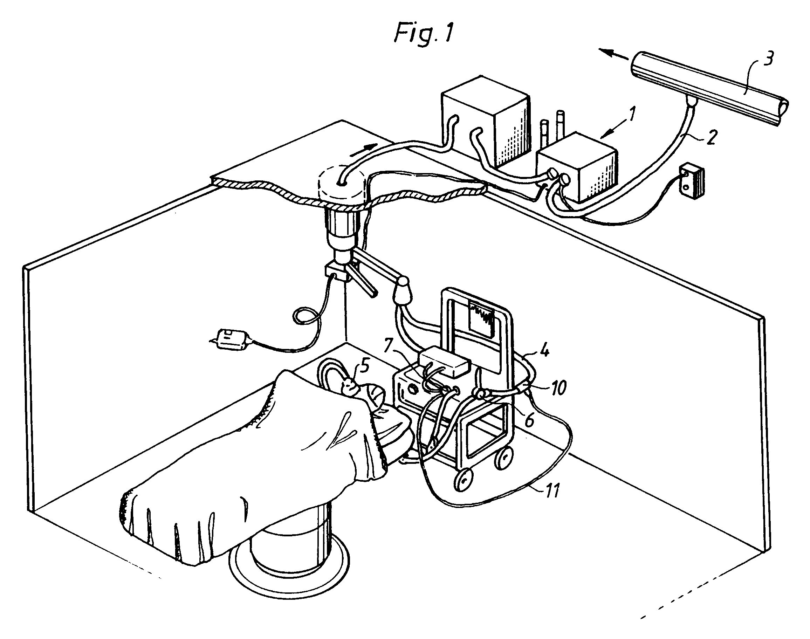

The invention will - in the following - be more closely described with references to the accompanying drawing, in which
Figure 1 is a perspective view schematically illustrating the build up of an evacuation system with an apparatus according to the invention,
Figure 2 is a larger scale and perspective view showing one connecting unit with attached tube nipple,
Figure 3 is a sectional view of the same connecting unit, and
Figure 4 is a larger scale and perspective view illustrating the connection at a control valve.
Figure 5 schematically illustrates the build up of an alternative embodiment of the system for reduction of the noise created by the evacuation device.

In the apparatus of this invention included a vacuum source in the form of a fan unit 1, which via an exhaust hose 2 is connectable to a permanent evacuation duct means 3 of a building alternatively in a room, e.g. an evacuation flue or valve for the general ventilation.

To the fan unit there is also connected a tube arrangement or suction channel 4, connected to an anaesthetic mask 5 via an intermediary section varying according to the type of anaesthetic system. The suction channel normally has a safety valve and flow meter 6 and also a resonance or damping chamber.

The mask 5 is supplied anaesthetic gas via a control and dosage unit generally designated 7 connected to an anaesthetic gas source and by a pressure and volume controller connected to the mask.

There are two main types of such control and dosage units, viz. the so called D system and the Circle system.

In order to facilitate the understanding of the invention, there is shown in Figure 4 a so called anaesthetic apparatus, i.e. a control and dosage unit 7 equipped with a so called breathing bubble or balloon 8 portioning out the fresh gas and also indicating if the breathing takes place in a normal way, and a so called reservoir 9 formed by a downwardly open pleated hose. The reservoir serves as a compensating vessel on the evacuation and secures that the pulsing exhalation may take place in the intended way in spite of the fact that the evacuation is essentially continuous. The reservoir is downwardly open also in order to be able to serve as a safety valve if a fault occurs between same and the mask so that no vacuum build up can take place. When - via a tube connected to the unit 7 - all exhaled air is removed, no vacuum build up can occur in the mask or within the respiratory system of the patient - the latter certainly resulting in grave danger - and pressure compensation will occur by air entering the reservoir 9 from below.

The control of the evacuation may also take place by means of a valve sensing the breathing sequence or the like means opening and closing the evacuation in pace with the inhalation and exhalation respectively.

The known masks 5 for removing and evacuation of excess and leaked out gas have the ability to prevent anaesthetic gas from polluting the environment both when the mask is kept applied towards the face of the patient and when held free, but they are not adapted or intended to take care of all exhaled air. Such air or part thereof will be removed and evacuated at so called Circle systems in the way earlier mentioned. And this in the case there is arrangements for such evacuation in the operation theatre.

In order to enable removal and evacuation of exhaled air also in places devoid of means for evacuation there is, according to this invention arranged at the suction channel 4 between the mask 5 and the fan unit 1, a connector 10, which - as shown in Fig. 4 - via a branch pipe or line 11 is connected to an evacuation socket at a conventional control unit 7.

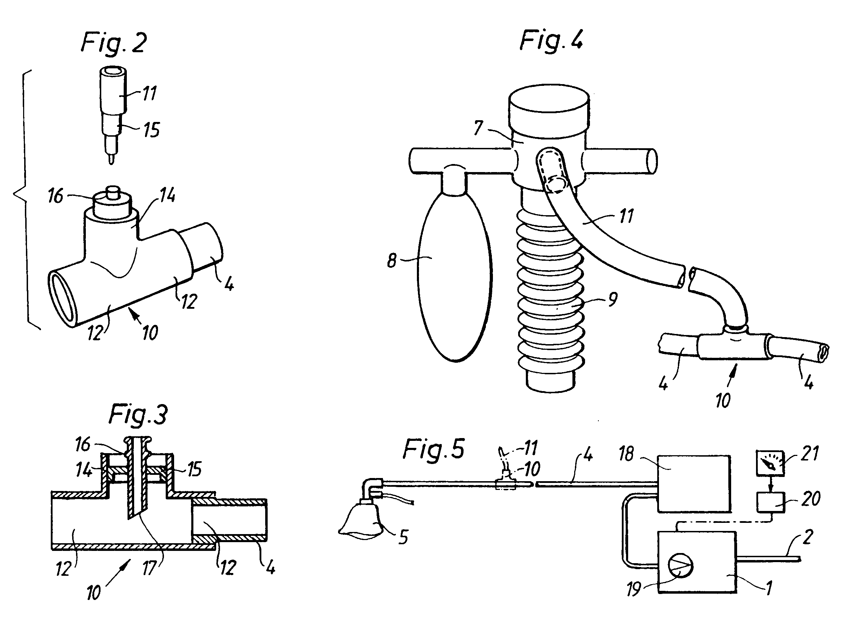

The connector 10 shown in Figs. 2 and 3, includes a T-pipe to the two mutually aligned connections 12 of which the suction channel 4 between the mask 5 and the vacuum unit 1 is connected. At the third connection 14 where the branch line 11 is connected there is a washer means 15 through which extends a nipple or nozzle means 16, the inner end of which is obliquely cut and extends essentially to the centre axis of the straight passage through the T-pipe. The size or area of the nipple 16 is adapted to the existing or expected vacuum inside the suction line 4. This vacuum is controlled, besides by the fan unit 1 by a counter pressure or resistance means, normally integrated in and/or constituted by the catching and removing parts of the mask 5 for leaked out or otherwise escaped gas, but may also be a separatly mounted resistance body as an end plug device insertable into the end of the tube. By size adaption of the nipple 16 and adjusting the vacuum inside the pipe 4, the desired evacuation or suction capacity may be created inside the evacuation tube 11. By changing the nipple and/or adjusting for a change in vacuum inside the channel 4, different evacuation capacities may be had if necessary.

Consequently, anaesthesia gas mixed exhaled air may be removed - through the branch line 11 connected to the socket at the control and dosage unit 7 - also with systems devoid of per se known pressurized or compressed air driven ejector means or the like means. This results in an improvement of the environmental situation inside also spartan surgical rooms so that they will be fully comparable with operation theatres having central evacuation for a low cost not possible earlier.

It should here be noted that the noise production caused by the extra suction connection is neglectable especially when compared with known compressed air driven ejectors which can have a very high noise level.

The evacuation capacity of the suction line 4 from the anaesthetic mask 5 to the fan unit 1 in a high-flow-low-pressure system of the kind herein described is or ought to be in the range of 15 - 39 m³/h (250 - 650 l/min) preferably 34,8m³/h (580 l/min) at large masks and from 16, 8- 26,4 m³/h (280 - 440 l/min) at medium to small masks. The capacity with the extra evacuation connection may be definitely lower and be of the range of 1,2 - 2,4 m³/h (20 - 40 l/min), preferably below 1,8 m³/h (30 l/min).

The heavy reduction of capacity in the evacuation or branch lines 11, is possible due to the pressure drop arising at the connector device 10 and due to resisting pressure caused at the input end of the channel, i.e. the gas collecting means of the mask 5 and the reduced area at the nipple menas 16. The reduction of suction capacity of course is biassed also by the difference between the areas of the suction pipe 4 and the nipple 16.

Concerning the suction velocity applies generally that for the intended purposes you ought to keep it within the limits given above, i.e. at about 15 - 39m³/h (250 - 650 l/min) for the anaesthetic mask suction. The capacity at the extra connection serving for the evacuation of the exhaled air may - as can be seen - be considerably lower and be of the range of 1,2 - 2,4 m³/h (20 - 40 l/min).

In order to keep the suction capacity within pre-set limits and adapt it to the mask size utilized, there is at the fan unit creating the necessary vacuum arranged a speed control device. The speed of the fan motor is controlled by means of an electronic control unit preferably built as an integrated circuit and with an automatically electronically controlled setable and vacuum sensing check or speed control device and a manually operated switch device for the setting of different effect steps and rotational speed areas, see figure 5.

## Claims

1. An apparatus for collecting and removing anaesthesy or analgesy gases escaping from an anaesthesia device in operation theatres or the like, comprising a vacuum source (1), a mask device (5) adapted to be applied to the face of a patient and communicating with an anaesthesia control and dosage unit (7) through at least one tube for supplying the patient with a mixture of anaesthesy or analgesy gas and air for removing gas and air mixture expired by the patient, respectively, the mask device (5) comprising collecting means connected to the vacuum source (1) through a suction channel (4) for removing anaesthesy or analgesy gas leaking out or otherwise escaping adjacent the mask (5), the suction channel (4) comprising means (10) for connecting a branch pipe or hose (11) communicating with the gas and air supply and removal tube,
**characterized** in that the means (10) connecting the branch pipe or hose (11) connected to the gas-air mixture supply and removing tube with the suction channel (4) is an ejector device (10), the ejector device being tee-shaped and including two aligned connections (12) coupled to the suction channel (4) and a third connection (14) connected with the branch pipe or hose (11), wherein the third connection (14) includes restricting means (15-16), creating in the branch pipe or hose (11) a vacuum capacity lower than that of the suction channel (4).

2. The apparatus of claim 1, wherein the restricting means (15-16) of the third connection (14) of the tee-shaped ejector device (10) includes a nipple means (16) projecting into a through flow path between the two aligned connections (12) coupled to the suction channel (4), the end of the nipple means (16) projecting into said path being obliquely cut.

3. The apparatus of claim 1, wherein a counter pressure or resistance means is mounted to the upstream end of the suction channel (4) to bias the vacuum in the suction channel.

4. The apparatus of claim 3, wherein the counter pressure or resistance means at the upstream end of the suction channel (4) is constituted of the means for collecting escaped gas at the mask device (5).

5. The apparatus of claim 3, wherein the counter pressure or resistance means is a separate resistance body mounted at the end of at the suction channel (4).

## Patentansprüche

1. Vorrichtung zum Sammeln und Entfernen von betäubenden oder schmerzstillenden Gasen, die aus einer Anästhesieeinrichtung in einem Operationssaal oder ähnlichem entweichen, mit einer Vakuumquelle (1), einer Maskeneinrichtung (5), die auf das Gesicht eines Patienten aufgesetzt wird und über mindestens ein Rohr mit einer Anästhesie-Steuer- und Dosiereinheit (7) zur Versorgung des Patienten mit einer Mischung aus betäubenden oder schmerzstillenden Gasen und Luft bzw. zum Entfernen von Gas und einer Luftmischung, die von dem Patienten ausgeatmet wird, verbunden ist, wobei die Maskeneinrichtung (5) Sammeleinrichtungen aufweist, die über einen Saugkanal (4) mit einer Vakuumquelle (1) zum Entfernen der betäubenden oder schmerzstillenden Gase, die aus der Nähe der Maske (5) austreten oder auf andere Weise entweichen, verbunden sind, und wobei der Saugkanal (4) Einrichtungen (10) zum Verbinden einer Zweigleitung oder eines Schlauches (11), die/der mit dem Rohr für die Gas- und Luftzu- und -abführung verbunden ist, aufweist,
dadurch gekennzeichnet, daß die Einrichtung (10), die die Zweigleitung oder den Schlauch (11) verbindet, die/der mit dem Rohr für Gas-Luftmischungsversorgung und -abführung verbunden ist, eine Ausblaseinrichtung (10) bildet, die T-fömig ist und zwei ausgerichtete Verbindungen (12) aufweist, die mit dem Saugkanal (4) verbunden sind, und eine dritte Verbindung (14) umfaßt, die mit der Zweigleitung oder dem Schlauch (11) verbunden ist, wobei die dritte Verbindung (14) Begrenzungseinrichtungen (15, 16) aufweist, die in der Zweigleitung oder dem Schlauch (11) eine Vakuumkapazität erzeugen, die geringer ist, als die des Saugkanals (4).

2. Vorrichtung nach Anspruch 1,
dadurch gekennzeichnet, daß die Begrenzungseinrichtung (15, 16) der dritten Verbindung (14) der T-förmigen Ausblaseinrichtung (10) einen Nippel (16) aufweist, der sich in einen Durchflußweg zwischen zwei ausgerichteten Verbindungen (12) erstreckt, die mit dem Saugkanal (4) verbunden sind, wobei das Ende des in den Weg hineinragenden Nippels (16) schräg geschnitten ist.

3. Vorrichtung nach Anspruch 1,
bei der eine Gegendruck- oder Widerstandseinrichtung an dem stromaufwärts gelegenen Ende des Saugkanals (4) montiert ist, um das Vakuum in dem Saugkanal vorzuspannen.

4. Vorrichtung nach Anspruch 3,
bei der die Gegendruck- oder Widerstandseinrichtung an dem stromaufwärtigen Ende des Saugkanals (4) durch die Einrichtung zum Sammeln des aus der Maske (5) ausgetretenen Gases gebildet ist.

5. Vorrichtung nach Anspruch 3,
bei der die Gegendruck- oder Widerstandseinrichtung ein getrennter Widerstandskörper ist, der an dem Ende des Saugkanals (4) montiert ist.

## Revendications

1. Un appareil pour recueillir et évacuer des gaz anesthésiants ou analgésiques s'échappant d'un dispositif d'anesthésie dans des théatres d'opérations ou analogues, comprenant une source de vide (1), un dispositif à masque (5) prévu pour être appliqué sur le visage d'un patient et communiquant avec une unité de commande et de dosage d'anesthésie (7) par l'intermédiaire d'au moins un tube permettant de fournir au patient un mélange de gaz anesthésiants ou analgésiques et d'air pour évacuer le mélange de gaz et d'air expiré par le patient, respectivement, le dispositif à masque (5) comprenant des moyens collecteurs reliés à la source à vide (1) par l'intermédiaire d'un canal d'aspiration (4) permettant d'évacuer les gaz anesthésiants ou analgésiques fuyant ou s'échappant autrement de manière adjacente au masque (5), le canal d'aspiration (4) comprenant des moyens (10) pour relier un conduit ou un tuyau souple de branchement (11) communiquant avec le tube d'amenée et d'évacuation des gaz et de l'air,
**caractérisé en ce que** les moyens (10) reliant le conduit ou le tuyau souple de branchement (11), relié au tube d'amenée et d'évacuation du mélange gaz-air, au canal d'aspiration (4) est un dispositif à éjecteur (10), le dispositif à éjecteur étant en forme de T et comprenant deux liaisons alignées (12) couplées au canal d'aspiration (4) et une troisième liaison (14) reliée au conduit ou tuyau souple de branchement (11), dans lequel la troisième liaison (14) comprend des moyens restricteurs (15-16) créant dans le conduit ou tuyau souple de branchement (11) une capacité de vide inférieure à celle du canal d'aspiration (4).

2. L'appareil de la revendication 1, dans lequel les moyens restricteurs (15-16) de la troisième liaison (14) du dispositif à éjecteur en forme de T (10) comprennent des moyens à manchon (16) faisant saillie dans un trajet d'écoulement traversant entre les deux liaisons alignées (12) couplées au canal d'aspiration (4), l'extrémité des moyens à manchon (16) faisant saillie dans ledit trajet étant coupée obliquement.

3. L'appareil de la revendication 1, dans lequel des moyens de contre-pression ou de résistance sont montés à l'extrémité amont du canal d'aspiration (4) pour pousser le vide dans le canal d'aspiration.

4. L'appareil de la revendication 3, dans lequel les moyens de contre-pression ou de résistance au niveau de l'extrémité amont du canal d'aspiration (4) sont constitués des moyens pour recueillir les gaz échappés au niveau du dispositif à masque (5).

5. L'appareil de la revendication 3, dans lequel les moyens de contre-pression ou de résistance sont un corps de résistance séparé monté à l'extrémité du canal d'aspiration (4).
